# EUROPÄISCHE PATENTANMELDUNG

(11) **EP 2 301 547 A1**
(43) Veröffentlichungstag der Anmeldung: **30.03.2011**
(21) Anmeldenummer: 10195559.9
(22) Anmeldetag: 06.07.2006
(51) Int. Cl.: A61K 31/506, A61P 17/02

(54) **Verwendung von Aktivatoren der löslichen Guanylatzyklase zur Förderung der Wundheilung**

(30) Priorität: 06.07.2005 DE 102005031575
(62) Teilanmeldung aus: 06762453.6
(71) Anmelder: Bayer Schering Pharma AG, 13353 Berlin (DE)
(72) Erfinder: Krahn, Thomas, 58135, Hagen (DE); Stasch, Johannes-Peter, 42651, Solingen (DE); Weimann, Gerrit, 51109, Köln (DE); Thielemann, Wolfgang, 42107, Wuppertal (DE); Stelte-Ludwig, Beatrix, 42489, Wülfrath (DE)

(57) **Zusammenfassung**

Die vorliegende Erfindung bezieht sich auf die Verwendung von Substanzen zur Herstellung eines pharmazeutischen Produktes / Medikamentes zur Förderung der Wundheilung.

## Beschreibung

Die vorliegende Erfindung bezieht sich auf die Verwendung von Substanzen zur Herstellung eines pharmazeutischen Produktes / Medikamentes zur Förderung der Wundheilung.

In den Prozess der Wundheilung bzw. die Regulierung der Heilung von geschädigtem Gewebe ist im Allgemeinen die Rekrutierung von Entzündungszellen involviert, gefolgt von den Fibroblasten, welche sich im Bereich der Wunde ansammeln. Kollagen und andere Bindegewebsbestandteile werden abgelagert und umgelagert, um das originale Bindegewebe (z.B. in Haut, Muskel, Bänder und Sehnen) wiederherzustellen. Deshalb ist es von einem großen klinischen Wert, Medikamente zu finden, die diesen Prozess nach chirurgischen Eingriffen, Verletzungen oder bei Störungen der Wundheilung fördern. Die Wundheilung ist gestört, d.h. verzögert oder gehemmt, z.B. bei Patienten mit systemischen Erkrankungen wie Diabetes, Nieren- oder Leberschäden, peripheren Gefäßerkrankungen und bei Patienten, welche Medikamente einnehmen, die die Wundheilung stören, z.B. Kortikosteroide, Immunsuppressiva und Substanzen zur Hemmung des Gefäßwachstums.

Jede Unterbrechung der anatomischen oder physiologischen Funktion eines Körpergewebes wird als Wunde bezeichnet. Das bedeutet, dass nicht nur ein Schnitt, der die Haut verletzt, eine Wunde ist. Auch wenn ein inneres Organ nicht mehr richtig funktioniert, ist das eine Wunde. Häufig werden diese physiologischen Wunden eher als Verletzung angesehen. Man unterscheidet daher auch zwischen offenen und geschlossenen Wunden. Zu den offenen Wunden gehören z.B. Schnitt-, Stich-, Platz-, Riss-, Schürf-, Biss- und Schusswunden sowie Hautablösungen und traumatische Amputationen. Zu den geschlossenen Wunden gehören z.B. Prellungen, Quetschungen, Distortionen, Verbrennungen, Erfrierungen, chemische Wunden durch Säuren oder Laugen, aktinische Wunden und Nekrosen (z.B. Gewebeschäden in Folge mangelhafter Durchblutung, z.B. Herz- und Hirninfarkte).

Ziel der Wundheilung ist die vollständige Regeneration, d.h. den Normalzustand des Gewebes wiederherzustellen. Die Regenerationsprozesse sind jedoch nicht immer in der Lage, diesen Zustand zu erreichen. Daher kommt es häufig nur zu einer unvollständigen Regeneration, z.B. wenn tiefer gelegene Hautschichten (z.B. Lederhaut) betroffen sind. Betroffen sind auch Gewebe, die sich kaum erneuern können (Herz- und Nervengewebe). Hier wird das verlorengegangene Gewebe meist durch funktionell minderwertiges Ersatzgewebe (Bindegewebe) aufgefüllt, das weniger durchblutet wird und weniger elastisch und funktionell ist als das ursprüngliche Gewebe. Dies wird als Narbe bzw. Narbengewebe bezeichnet. Um die maximale Funktionalität wiederzuerlangen, kann es also auch eine Ziel sein, funktionelles Gewebe (z.B. Muskelzellen) in seiner Regeneration gegenüber Bindegewebe (Fibroblasten) zu fördern.

Es ist bekannt, dass Mechanismen (z.B. NO freisetzende Substanzen), die zu einer Erhöhung des intrazellulären Botenstoffes cGMP führen, auch zu einer Verbesserung der Wundheilung beitragen können (WO 96/08966-A1, Murrell).

Die Aktivierung (durch Agonisten hervorgerufen) der löslichen Guanylatzyklase führt zu einem Anstieg des intrazellulären Botenstoffes cGMP. Überraschenderweise ist jetzt gefunden worden, dass die unten aufgeführten erfindungsgemäßen Aktivatoren der löslichen Guanylatzyklase, Verbindungen II-IVa, für die Herstellung von pharmazeutischen Substanzen / Medikamenten für die Förderung der Wundheilung insbesondere am Menschen geeignet sind.

Verbindung (II) entspricht der folgenden Formel:

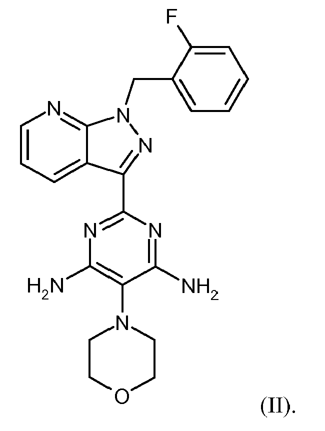

Verbindung (II), deren Herstellung und Verwendung als Arzneimittel wurden bereits in der WO 00/06569 offenbart.

Verbindung (III) entspricht der folgenden Formel:

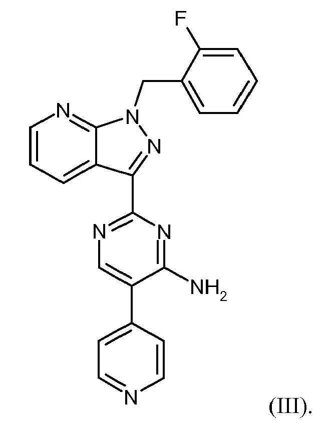

Verbindung (III), deren Herstellung und Verwendung als Arzneimittel wurden bereits in der WO 00/06569 und WO 02/42301 offenbart.

Verbindung (IV) entspricht der folgenden Formel:

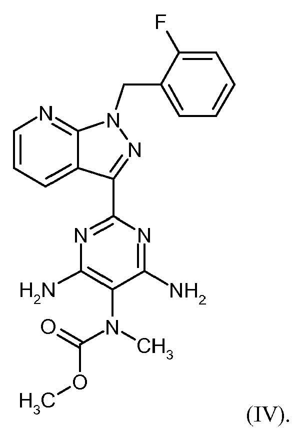

Verbindung (IV), deren Herstellung und Verwendung als Arzneimittel wurden bereits in der WO 00/06569 und WO 03/095451 offenbart.

Verbindung (IVa) entspricht der folgenden Formel:

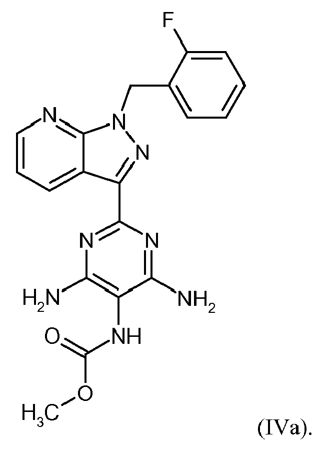

Verbindung (IVa), deren Herstellung und Verwendung als Arzneimittel wurden bereits in der WO 00/06569 und WO 03/095451 offenbart.

Gegenstand der vorliegenden Erfindung ist die Verwendung von Verbindungen der Formeln (II-IVa) und deren Salze, Hydrate, Hydrate der Salze für die Herstellung eines Medikamentes für die Förderung der Wundheilung.

Ein weiterer Gegenstand der vorliegenden Erfindung ist die pharmazeutische Zusammensetzung, welche mindestens eine Verbindung der Formeln (II-IVa) mit einem pharmazeutisch akzeptablen Träger enthält, jeweils für jeden der zuvor diskutierten therapeutischen Effekte. Die Zusammensetzung kann allein oder in Kombination mit mindestens einer weiteren Substanz, z.B. einer stabilisierenden Substanz, gegeben werden.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal (topisch), conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung der Formel (II-IVa), üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, pro Tag Mengen von etwa 0.01 bis 5000 mg/kg, vorzugsweise etwa 0.5 bis 1000 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Bevorzugter Gegenstand der vorliegenden Erfindung ist die topische Anwendung einer pharmazeutischen Formulierung.

Ein weiterer Gegenstand der vorliegenden Erfindung sind Suspensionen und Salben zur topischen Anwendung, die mindestens eine der Verbindungen der Formeln (II-IVa) enthalten. Zu weiteren topischen Zubereitungen der Erfindung gehören Lösungen, Sprays, Lotionen, Gele, Cremes, Pulver, Pudersprays, Pasten, Emulsionen, Schäume und Stifte, die mindestens einen Wirkstoff der Formel (II-IVa), gegebenenfalls auch mehrere Wirkstoffe enthalten.

Die topische Applikation eines Wirkstoffs der Formel (II-IVa) erfolgt auch in Form von Pflastern, Sprühpflastern, Okklusivverbänden, Umschlägen und gesteuerten Abgabesystemen. In diesen Zubereitungen können die Wirkstoffe in gelöster oder suspendierter Form enthalten sein.

Salben enthalten als Grundlage Kohlenwasserstoffgele, Lipogele, Absorptionsgrundlagen, W/O-Salbengrundlagen, Mischemulsionen oder Polyethylenglykole.

Cremes enthalten O/W-Grundlagen.

Pasten enthalten neben einer Salben- oder Cremegrundlage hohe Anteile pulverförmiger Bestandteile wie zum Beispiel Zinkoxid, Talk, Stärke oder Titandioxid.

Gele enthalten Lösungsmittel wie Wasser, Ethanol, Isopropanol oder Propylenglykol und werden unter Verwendung von Gelbildnern wie Celluloseethern, Alginaten, Polyacrylaten, Bentonit, Gelatine, Tragant, Polyvinylpyrrolidon oder Polyvinylalkohol hergestellt. Auch die Verwendung lipophiler Gelgrundlagen oder von Mikroemulsionen ist möglich.

Puder enthalten pulverförmige Zusatzstoffe wie Stärke, Stearate, Siliciumdioxid, Ton, Magnesiumcarbonat, Talk, Cellulose, Zinkoxid und insbesondere Lactose.

Allen Zubereitungen können Stabilisatoren, Antioxidantien, Konservierungsmittel, Feuchthaltemittel, Rückfetter, Lösungsmittel oder Hilfsstoffe zur Verbesserung von Penetration und Wirksamkeit zugesetzt werden.

Beispiele von Penetrationsverbesserern sind Propylenglykol, Polyethylenglykol, Dimethylsulfoxid, Decylmethylsulfoxid, Azone, N-Methylpyrrolidon, Diethyltoluamid, Ethanol, Isopropylmyristat, Isopropylpalmitat, Ölsäure und ihre Ester, mittelkettige Triglyceride, Dimethylisosorbit, 2-Octyldodecanol, verzweigtkettige Fettsäureester, Benzylalkohol, Harnstoff, Salicylate und Tenside.

Als eine weitere für die topische Anwendung geeignete pharmazeutische Formulierung kann ein gemäss dem Stand der Technik aus der pharmazeutischen Technologie bekanntes Trägersystem auf der Basis von Liposomen verwendet werden. Dieses sollte insbesondere die Penetration von Lipiden in die Epidermis verbessern. Solche Liposomendispersionen mit verschiedenen Einschlussverbindungen und Phospholipiden als Doppelschichtmembranbildner oder sogenannte leere Liposomen ohne Einschlussverbindungen sind in zahlreichen Publikationen beschrieben und bereits klinisch erprobt worden. Die Herstellung erfolgt z.B. durch Behandlung einer wässrigen Phospholipiddispersion mit Ultraschall, Dispersion von Phospholipiden mit Tensiden in wässriger Phase, Auflösen von Phospholipiden in organischen Lösungsmitteln, Entfernen des Lösungsmittels durch Lyophilisierung und Dispersion des Rückstandes in wässriger Phase, Infusionsmethoden oder Umkehrphasenverdampfung. Bevorzugte pharmazeutische Formulierungen für die topische Anwendung sind feindisperse Systeme auf der Basis von Lipidgemischen.

Aus dem Dokument WO 96/37192 sind pharmazeutische oder kosmetische Zusammensetzungen bekannt, die in Kombination mit einem Sphingolipid oder Glycolipid (Ceramid) folgendes enthalten: einen Partialfettsäureester des Polyoxyethylensorbitans, ein Phospholipid, ein Triglycerid und einen therapeutischen Wirkstoff, in Wasser (und eventuell Alkanol) als Trägerflüssigkeit. Aus EP 0852941 sind Zusammensetzungen bekannt, bei denen sich die Wirkstoffe durch ein ähnliches Hilfsstoffgemisch, vom Typ Partialfettsäureester des Polyoxyethylensorbitans, Phospholipid und Ethanol, auch in Wasser unlösliche, flüssige bis hochviskose, ölige oder jedenfalls in Öl lösliche Wirkstoffe, z.B. fettlösliche Vitamine, therapeutische Öle oder Lichtschutzsubstanzen, in Form einer Dispersion von Nanopartikeln (Nanodispersion) solubilisieren lassen. Die in dieser Offenbarung beschriebenen pharmazeutischen Formulierungen enthaltend Verbindungen der Formeln (II-IVa) gelten als bevorzugter Gegenstand der vorliegenden Erfindung.

Die Formulierungen können dabei entsprechend des Eingriffes aktive Substanz zwischen 0.1 und 99% Wirkstoff enthalten, in geeigneter Weise 25-95% bei Tabletten und Kapseln und 1-50% bei flüssigen Formulierungen, d.h. der Wirkstoff sollte in Mengen vorliegen, die ausreichend sind, den angegebenen Dosierungsspielraum zu erreichen.

Ein weiterer Gegenstand der vorliegenden Erfindung ist die Verwendung einer Kombination von einer oder mehreren der erfindungsgemäßen Verbindungen (II-IVa) mit einer oder mehreren anderen Substanzen. Geeignete Kombinationspartner sind zum Beispiel Substanzen, die das Gefäßwachstum fördern. Beispielhaft und bevorzugt werden in diesem Zusammenhang cGMP erhöhende Substanzen, Adenosinagonisten und Wachstumsfaktoren genannt.

Ein weiterer Gegenstand der vorliegenden Erfindung ist die Verwendung einer Kombination von einer oder mehreren der erfindungsgemäßen Verbindungen (II-IVa) mit einer oder mehreren Substanzen ausgewählt aus der Gruppe der Medikamente zur Förderung der Durchblutung, zur Schmerzstillung oder auch der Antibiotika.

### Experimenteller Teil:

### 1. Beeinflussung der Migration von Glattmuskelzellen in vitro durch Gabe eines Aktivators der löslichen Guanylatzyklase:

Glatte Muskelzellen der humanen Koronararterie werden auf einer 6-Lochplatte ausgesät (1.5 x 10⁵ Zellen/well) (Clonetics) und für 48 h in SmGM2-Medium (Clonetics) kultiviert. Vor dem Gebrauch der Platten werden die Vertiefungen mit Vitronektin (50 ng/cm²; Gibco BRL) beschichtet. Die Hälfte des konfluenten Zell-Monolayers wird mit Hilfe eines Zellschabers entfernt (wounding). In dem zellfreien Bereich bleibt hierbei die Vitronektin-Beschichtung zu mindestens 50% erhalten. Für den Versuch wird, soweit nicht anders erwähnt, das Kulturmedium durch MCDB-131/0.2% BSA (Gibco/BRL) Medium ersetzt.

Die Prüfsubstanzen werden in verschiedenen Konzentrationen zugefügt und die Migrationsstrecke in den zellfreien Bereich mit Hilfe eines Mikroskops über 24 h und 48 h verfolgt. Jeder Messpunkt repräsentiert das Mittel aus vier gemessenen Regionen. PDGF, als potenter chemotaktischer Faktor für SMC, wird als Positivkontrolle verwendet (1 nM, 10 nM, R&D systems). Zur Inhibition der Integrin-induzierten Migration wird das RGD-Tripeptid (Bachem) eingesetzt. Zur Bestimmung der antagonistischen Aktivität der Prüfsubstanzen werden die Zellen in Gegenwart der Substanz mit 1 nM bzw. 10 nM PDGF stimuliert und die Migrationsstrecken in Korrelation zu den PDGFinduzierten Migrationsstrecken bestimmt.

Repräsentative Ergebnisse aus diesem Test sind in der folgenden Tabelle wiedergegeben:

**Tabelle. Inhibition der horizontalen Migration von Glattmuskelzellen der humanen Koronararterie (48 h Inkubationszeit)**

| **Verbindung** | **IC₅₀ [nM]** |
|---|---|
| IV | 10 |
| IVa | 30 |

Die Verbindungen II-IVa zeigen, dass Aktivatoren der löslichen Guanylatzyklase geeignet sind, die Wundheilung zu fördern.

### 2. Formulierung der Wirkstoffe

### Ophthalmologische topische Anwendung

Die Wirkstoffe werden zur Bildung einer 2 Gew.-% Suspension mit Liquifilm^{©} Augentropfen (Allergan, Ettlingen, Deutschland) (1 ml enthaltend Polyvinylalkohol 14 mg, sowie als Hilfsstoff Chlorbutanol x ½ H₂O 5.25 mg, Natriumchlorid) versetzt und in einem Glasröhrchen 15 min in einem Ultraschallbad bei 15°C sonifiziert.

### Dermatologische topische Anwendung

### 1) Alkoholische Suspension

Die Wirkstoffe werden zur Bildung einer 2 Gew.-% Suspension mit 30 Gew.-% Isopropylmyristat/70 Gew.-% Ethanol versetzt und in einem Glasröhrchen 15 min in einem Ultraschallbad bei 15°C sonifiziert.

### 2) Streichfähige Zubereitungen

Zur Herstellung der Salbenzubereitung wird Wollwachsalkoholsalbe nach DAB 9 eingesetzt. Der Wirkstoff wird in einer aufgeschmolzenen Mischung aus 0.5 Gewichtsteilen Cetylstearylalkohol (Qualität nach DAB 9), 6 Gewichtsteilen Wollwachsalkohol (Qualität nach DAB 9) und 93.5 Gewichtsteilen weißem Vaselin (Qualität nach DAB 9) suspendiert. Die Mischung wird bis zum Abkühlen auf Raumtemperatur (etwa 21°C) gerührt.

Zur Herstellung einer sogenannten "PEG-Isogel"-Zubereitungwird der Wirkstoff in der Schmelze einer Mischung aus niedermolekularem Polyethylenglycol (PEG) und höhermolekularem PEG suspendiert bzw. gelöst. Nach Suspendieren bzw. Lösen des Wirkstoffs in der Schmelze wird die Zubereitung bis zum Erkalten auf Raumtemperatur gerührt.

Niedermolekulares PEG ist bei Raumtemperatur (etwa 21°C) flüssig, höhermolekulares PEG ist bei Raumtemperatur fest (schneidbar, wachsartig).

Die Mischungsverhältnisse der beiden PEG-Typen richten sich nach erforderlicher Viskosität der Zubereitung und nach den mittleren Molekulargewichten der eingesetzten PEG-Typen.

Als Beispiele können Zubereitungen aus 6.5 Gewichtsteilen PEG 400 und 1 Gewichtsteil PEG 4000 oder 7 Gewichtsteilen PEG 400 und 2 Gewichtsteilen PEG 6000 verwendet werden. Mischungsbereiche bewegen sich von 1:1 1 bis 10:1 (jeweils Gewichtsteile niedermolekulares PEG zu höhermolekularem PEG). Bevorzugt ist der Bereich von 2:1 bis 8:1, besonders bevorzugt der Bereich von 3:1 bis 7:1.

Als weitere Gelzubereitung wird ein Polyacrylatgel (sogenanntes "Carbogel"), welches aus 1 Gewichtsteil Carbopol 974 P NF (Hersteller Fa. BF Goodrich, USA), 5 Gewichtsteilen Isopropylalkohol, 5 Gewichtsteilen Natronlauge (5 Gew.-%) und 89 Gewichtsteilen Wasser besteht, eingesetzt. Zur Herstellung der Zubereitung wird das Carbopol mit dem Isopropanol angerieben, der Wirkstoff zugesetzt und die Mischung in Wasser dispergiert. Die Natronlauge wird schrittweise unter Rühren zugegeben. Es bildet sich ein Gel.

## Patentansprüche

1. Verwendung von Verbindungen der Formeln (II-IVa) 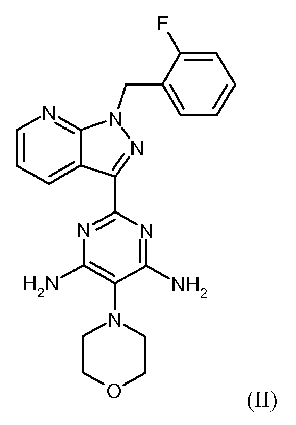 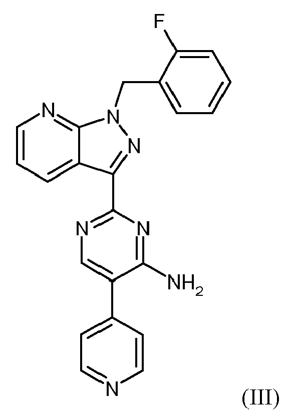 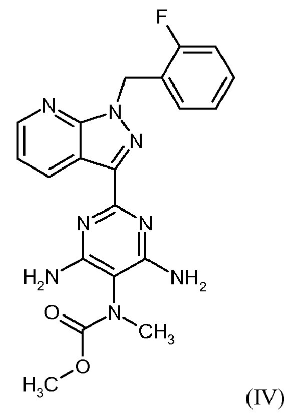 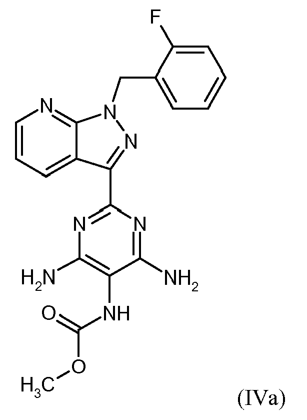 und deren Salze, Hydrate, Hydrate der Salze für die Herstellung eines Medikamentes für die Förderung der Wundheilung.

2. Verwendung gemäß Anspruch 1, worin das Medikament für eine topische Darreichungsform verwendet wird.

3. Verwendung gemäß Anspruch 1, worin das Medikament subkutan oder intramuskulär gegeben wird.

4. Verwendung gemäß einem der Ansprüche 1 bis 3, worin das Medikament vorbeugend angewendet wird.

5. Verwendung gemäß einem der Ansprüche 1 bis 4, worin das Medikament zur Behandlung von Wunden bei Diabetikern angewendet wird.

6. Pharmazeutische Zusammensetzung für die Förderung der Wundheilung, welche mindestens eine Substanz gemäß Anspruch 1 enthält.

7. Pharmazeutische Zusammensetzung gemäß Anspruch 6, die zusätzlich ein Medikament zur Förderung der Durchblutung, zur Schmerzstillung oder auch Antibiotika enthält.
